# EUROPÄISCHE PATENTSCHRIFT

(11) **EP 0 871 608 B1**
(45) Veröffentlichungstag und Bekanntmachung des Hinweises auf die Patenterteilung: **05.04.2000**
(21) Anmeldenummer: 96946110.2
(22) Anmeldetag: 20.12.1996
(51) Int. Cl.: C07C 229/16, C07C 229/46, A61K 49/00

(54) **Monofunktionalisierte EDTA-, DTPA- und TTHA-Derivate und deren Verwendung zur Herstellung von Mitteln für die medizinische Diagnostik oder Therapie**
Monofunctionalised EDTA-, DTPA- and TTHA-Derivatives and their use for the preparation of a diagnoctic or therapeutic medicine
Dérives d'EDTA, de DTPA et de TTHA et leur utilisation pour la préparation du médicament diagnostique ou thérapeutique

(30) Priorität: 04.01.1996 DE 19601060
(43) Veröffentlichungstag der Anmeldung: 21.10.1998
(73) Patentinhaber: SCHERING AKTIENGESELLSCHAFT, 13353 Berlin (DE)
(72) Erfinder: PLATZEK, Johannes, D-12621 Berlin (DE); MARESKI, Peter, D-13409 Berlin (DE); NIEDBALLA, Ulrich, D-14195 Berlin (DE); RADÜCHEL, Bernd, D-13465 Berlin (DE)
(86) Internationale Anmeldenummer: DE9602476
(87) Internationale Veröffentlichungsnummer: WO9725305

(56) Entgegenhaltungen:
- EP-A- 0 331 616
- GB-A- 1 076 146
- GB-A- 1 453 694
- US-A- 5 514 810
- JOURNAL OF MEDICINAL CHEMISTRY, Bd. 39, Nr. 18, 30.August 1996, WASHINGTON US, Seiten 3451-3460, XP000197344 YASUSHI ARANO ET AL.: "Reassessment of diethylenetriaminepentaacetic acid....."

## Beschreibung

Die Erfindung betrifft neue monofunktionalisierte EDTA-, DTPA- und TTHA-Derivate und deren Verwendung zur Herstellung von pharmazeutischen Mitteln.

Metallkomplexe, bestehend aus einem offenkettigen Chelatbildner (Liganden) und Metallionen, sind bekannte pharmazeutische Mittel, die vielfältige Anwendungen in der medizinischen Diagnostik und der Therapie von Schwermetallvergiftungen haben. So wurde u.a. das Megluminsalz des Gadolinium (III)-Komplexes der Diethylentriaminpentaessigsäure (DTPA) unter dem Handelsnamen Magnevist® als Kontrastmittel für die MR-Tomographie zugelassen.

Für die Entwicklung neuer Präparate ist es häufig erforderlich, ein oder mehrere zur Chelatbildung befähigte Liganden, wie z. B. Ethylendiamintetraessigsäure (EDTA), Diethylentriaminpentaessigsäure (DTPA) oder Triethylentetraaminhexaessigsäure(TTHA) an ein anderes Molekül zu binden. So ist es z.B. möglich, einen DTPA Rest an einen monoklonalen Antikörper zu binden, der seinerseits eine Spezifität gegenüber Tumoren aufweist, um diesen, mittels eines komplexierten radioaktiven Metallions zu markieren und somit zu detektieren. Ein solches Verfahren wurde z.B. von Kuhlmann und Steinsträsser (Nucl. Med. Biol. 15 (1988) pp. 617-627) beschrieben. Weiterhin ist es möglich, mehrere Komplexbildner-Reste an einen polymeren Grundkörper zu binden und somit die Anzahl der Metallionen, die als kontrastgebende Komponente im Molekül vorhanden sind, zu erhöhen. Verbindungen dieses Typs werden beispielsweise in der europäischen Patentanmeldung EP 331616 beschrieben.

Für die Herstellung derartiger Verbindungen werden in jedem Fall entsprechend vorbereitete monofunktionalisierte Komplexbildnerderivate benötigt. So benutzen z.B. Kuhlmann und Steinsträsser das von Hnatowich erstmals beschriebene Bisanhydrid der DTPA (Int.J.Radiat.Isot. 33 (1982), pp. 327 - 332). Das Hauptproblem bei der Verwendung des Bisanhydrides der DTPA ist jedoch dessen schlechte Löslichkeit in organischen Solvenzien, was eine Fülle weiterer Probleme nach sich zieht.
Ein weiteres Problem der Anwendung des Bisanhydrides der DTPA zur Verküpfung von DTPA an aminhaltige Moleküle ist die unvermeidlich auftretende Vernetzung, wie durch eine kritische Untersuchung von Maisano et al. (Bioconj. Chem. **3** (1992), pp. 212-217) belegt werden konnte. Die Erfinder der europäischen Patentanmeldung EP 331616 schlagen aus diesem Grunde die Verwendung eines aus dem Bisanhydrid gewonnenen Monoanhydrids, welches noch einen Ethylester trägt (siehe Beispiel 13a), vor. Dieses Derivat zeigt zwar eine bessere, aber noch immer unbefriedigende, Löslichkeit und weist gleichzeitig mit der Ethylestergruppierung einen Rest auf, der leicht unbeabsichtigt zu verseifen und daher problematisch zu handhaben ist. Darüber hinaus ist die Synthese aufwendig, unter anderem wegen der notwendigen chromatographischen Trennungen.

Die partielle alkalische Verseifung von DTPA-Pentamethylester, wie sie in US 5252317 beschrieben wird, führt stets zu Produktgemischen, da die Verseifung naturgemäß statistisch verläuft.

Aufgabe der vorliegenden Erfindung war es daher, neue Derivate der EDTA , DTPA und der TTHA zur Verfügung zu stellen, die eine selektive Ankoppelung an weitere Moleküle ermöglichen. Darüber hinaus sollen sie leicht herstellbar sein und eine bessere Löslichkeit als die bekannten Derivate aufweisen.Gleichzeitig sollen sie Carbonsäureschutzgruppen tragen, die auf Wunsch leicht und selektiv abspaltbar sind, die aber nicht im Verlaufe weiterer Manipulationen am Molekül unbeabsichtigt abgespalten werden.

Diese Aufgabe wird durch die vorliegende Erfindung gelöst, insbesondere durch die neuen Stoffe der Patentansprüche 1 und 2 und das Herstellungsverfahren des Patentanspruchs 6. Vorteilhafte Ausführungsformen der Erfindung sind Gegenstand der Unteransprüche.

Gegenstand der vorliegenden Erfindung sind daher Verbindungen der allgemeinen Formel I 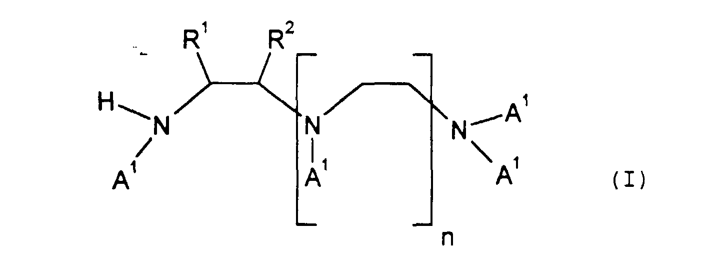 worin
- n: für die Zahlenwerte 0, 1 oder 2 steht,
- A¹: für den Rest -CH₂CO₂^{t}Bu steht,
- R¹ und R²: entweder jeweils für Wasserstoff oder gemeinsam für -(CH₂)ₘ- stehen, worin m die Zahlenwerte 3 bis 6 annehmen kann,
mit der Maßgabe, daß R¹ und R² nur dann gemeinsam für -(CH₂)ₘ- stehen, wenn n für den Wert 0 steht.

Die Formel I betrifft EDTA-(n=0) , DTPA-(n=1) und TTHA-(n=2) Derivate, bei denen jeweils eine endständige Essigsäurefunktion durch Wasserstoff substituiert ist,bevorzugt aber DTPA Derivate. Die Essigsäurefunktionen in den Gruppen A¹ sind in Form ihrer Tert.-butylester ausgelegt. Diese weisen den Vorteil auf, daß sie bei Bedarf durch Behandlung mit Trifluoressigsäure leicht und selektiv abspaltbar sind. Die Verbindungen sind in organischen Lösungsmitteln gut löslich, so daß sie einfach an eine Vielzahl von Molekülen geknüpft werden können. Nach Abspaltung der Säureschutzgruppen können dann Metallkomplexe gebildet werden, die für die medizinische Anwendung in Diagnostik und/oder Therapie geeignet sind.

Die Erfindung betrifft weiterhin Verbindungen der allgemeinen Formel II 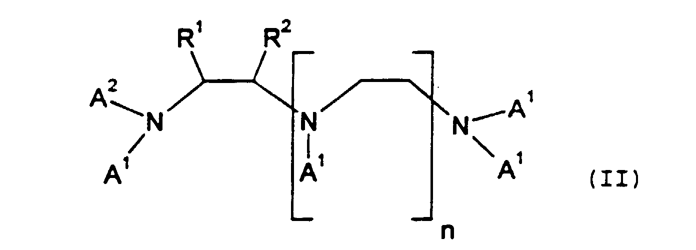 worin
- n: für die Zahlenwerte 0, 1 oder 2 steht,
- A¹: für den Rest -CH₂CO₂^{t}Bu steht,
- A²: für den Rest -CH₂CO₂H steht,
- R¹ und R²: entweder jeweils für Wasserstoff oder gemeinsam für -(CH₂)ₘ- steht, worin m die Zahlenwerte 3 bis 6 annehmen kann,
mit der Maßgabe, daß R¹ und R² nur dann gemeinsam für -(CH₂)ₘ- stehen, wenn n für den Wert 0 steht.

Die Verbindungen der allgemeinen Formel II betreffen gleichfalls EDTA, DTPA und TTHA Derivate. Von den in den Verbindungen vorhandenen Essigsäuregruppen ist eine als freie Säuregruppe vorhanden, der Rest wiederum als Tertiärbutylester ausgelegt. Auch diese Verbindungen weisen eine hervorragende Löslichkeit in organischen Solvenzien auf, sodaß sie ähnlich den Verbindungen der allgemeinen Formel I einfach und sicher an eine Vielzahl von Molekülen geknüpft werden können. Die Gruppen R¹ und R² können bei EDTA Derivaten auch gemeinsam für einen Oligomethylenrest stehen, so daß gemeinsam mit den beiden Kohlenstoffatomen des backbones ein Cycloalkylring, bevorzugt ein Cyclohexylring, entsteht.

Die Erfindung betrifft weiterhin ein Verfahren zur Herstellung von Verbindungen der allgemeinen Formel I 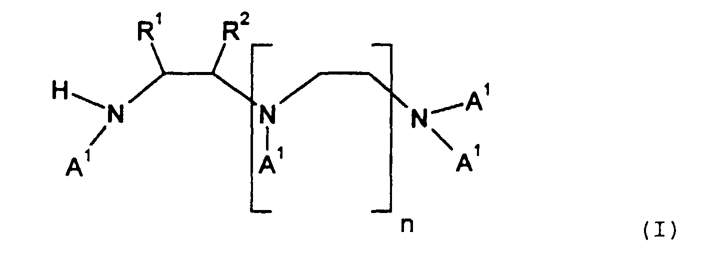 worin
- n: für die Zahlenwerte 0, 1 oder 2 steht,
- A¹: für den Rest -CH₂CO₂^{t}Bu steht,
- R¹ und R²: entweder jeweils für Wasserstoff oder gemeinsam für -(CH₂)ₘ- steht, worin m die Zahlenwerte 3 bis 6 annehmen kann,
mit der Maßgabe, daß R¹ und R² nur dann gemeinsam für -(CH₂)ₘ- stehen, wenn n für den Wert 0 steht,
dadurch gekennzeichnet daß, eine Verbindung der allgemeinen Formel III 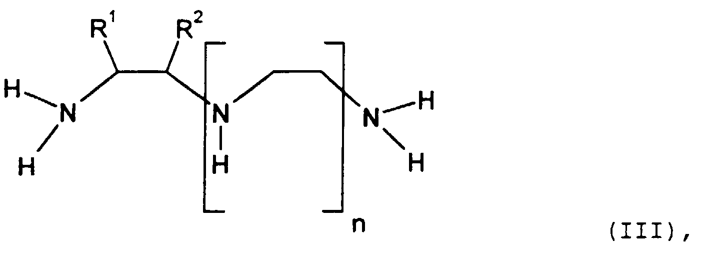 worin R¹, R² und n die oben genannten Bedeutungen besitzen mit einem Schutzgruppenreagenz zu einer Verbindung der allgemeinen Formel IV 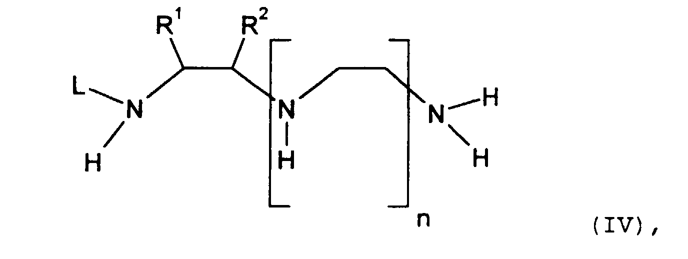 worin
R¹, R² und n die oben genannten Bedeutungen besitzen und
L für eine Trifluoracetyl- oder eine Benzylgruppe steht
umgesetzt wird, diese mit Verbindungen der allgemeinen Formel V

X-CH₂-COO^{*t*}Bu (V)

worin X für Chlor, Brom oder Jod steht,
umgesetzt wird
und die so erhaltene Verbindung durch Abspaltung der Schutzgruppe L in die Verbindung der allgemeinen Formel I überführt wird.

Die Einführung der Schutzgruppen L geschieht dergestalt, daß entweder die Trifluoracetylschutzgruppe dadurch eingeführt wird, daß das als Edukt eingesetzte Amin mit Trifluoressigsäureethylester umgesetzt wird, oder daß die Benzylschutzgruppe derartig eingeführt wird, daß zunächst das Amin mit Benzaldehyd umgesetzt wird, und das resultierende Produkt mit Natriumborhydrid reduziert wird. Das dadurch in jedem Fall entstehende Zwischenprodukt der allgemeinen Formel IV wird anschließend mit Halogenessigsäuretertiärbutylester umgesetzt. Nach Abspaltung der Schutzgruppe L durch alkoholische Ammonolyse der Trifluoracteylgruppe oder durch katalytische Hydrierung der Benzylgruppe wird direkt die gewünschte Verbindung der allgemeinen Formel I erhalten. Genaue Bedindungen und Vorgehensweisen sind in den Beispielen offenbart. Der Fachmann auf dem Gebiet verfügt über das notwendige Fachwissen, um die Synthese zu modifizieren und sie damit seinen jeweiligen Bedürfnissen anzupassen.

Gegenstand der Erfindung ist ebenfalls ein Verfahren zur Darstellung der Verbindungen der allgemeinen Formel II , daß dadurch gekennzeichnet ist, daß die Verbindungen der allgemeinen Formel I mit Halogenessigsäure (Chlor-, Brom- oder Iodessigsäure) umgesetzt wird. Dieses Verfahren kann auch derart ausgestaltet sein, daß die Umsetzung mit einem Halogenessigsäureester erfolgt, wobei als Estergruppe eine Gruppe gewählt wird, die ohne Beeinflussung der Tertiärbutylesterfunktionen verseift werden kann. Es ist bespielsweise möglich, eine Verbindung der allgemeinen Formel I mit Halogenessigsäurebenzylester umzusetzen. Aus der entstehenden Verbindung kann der Benzylrest durch katalytische Hydrierung (Katalysator z.B. Palladium auf Aktivkohle) entfernt werden, wobei die Verbindung der allgemeinen Formel II erhalten wird. Analog zu dieser Variante kann auch eine Umsetzung einer Verbindung der allgemeinen Formel I mit Halogenessigsäuremethylester und die folgende Abspaltung des Methylrestes mit einer äquivalenten Menge Base erfolgen.

Die Verbindungen der allgemeinen Formeln I und II können in vielfältiger Weise zur Herstellung von Mitteln für die medizinische Diagnostik oder Therapie eingesetzt werden. Verwendung finden sie insbesondere für die Herstellung von Mitteln für die MRI-, für die Röntgen- oder für die Radiodiagnostik. Verwendung finden sie aber auch für die Herstellung von Mitteln für die Radiotherapie. Details der genannten Verwendungsarten können z.B aus der EP 430863 entnommen werden.
Die Verbindungen können auch verwendet werden für die Herstellung von Mitteln, die als Antidot bei Schwermetallvergiftungen wirken.
Die Erfindung betrifft daher auch die genannten Verwendungen zur Herstellung pharmazeutischer Mittel.

Die nachfolgenden Beispiele sollen den Erfindungsgegenstand beschreiben, ohne ihn auf diese beschränken zu wollen:

### Beispiel 1

### 6, 9-Bis(t-butoxycarbonylmethyl)-3-carboxymethyl-3,6,9-triazaundecandicarbonsäure-di-t-butylester

### a) 1-Trifluoracetyl-1,4,7-triazaheptan

In 300 ml absolutem Tetrahydrofuran werden unter Abdeckung mit Stickstoff 21,6 ml (200 mmol) 1,4,7-Triazaheptan gelöst. Man kühlt auf 0°C und tropft dann 26,2 ml (220 mmol) Trifluoressigsäureethylester in 400 ml absolutem Tetrahydrofuran gelöst dazu. Nach Rühren über Nacht bei Raumtemperatur wird bei 35 - 40°C Badtemperatur im Vakuum eingeengt.
Ausbeute: 39,6 g (99,4 % d. Th.)

Nach Dünnschichtchromatographie (Kieselgel,
Dioxan/Wasser/Ammoniak= 10/1/1) besteht das Reaktionsprodukt aus einem Gemisch Monoamid und Diamid im Verhältnis 9:1.

| Elementaranalyse: C₆H₁₂F₃N₃O | | | | |
|---|---|---|---|---|
| ber. | C 36,18 | H 6,07 | F 28,62 | N 21,10 |
| gef. | C 35,51 | H 5,57 | F 30,65 | N 19,78 |

### b) 6,9-Bis(t-butyloxycarbonylmethyl)-3-trifluoracetyl-3,6,9-triaza-undecandicarbonsäure-di-t-butylester

In 450 ml absolutem Dimethylformamid werden 39,6 g (178,9 mmol Monoamid) des unter la) hergestellten Amidgemisches gelöst. Man versetzt mit 138,21 g (1000 mmol) gepulvertem Kaliumcarbonat, tropft dann unter Eiskühlung 195,06 g (1 mol) Bromessigsäure-t-butylester dazu und vervollständigt die Reaktion durch Rühren über Nacht bei Raumtemperatur. Man verdünnt mit Diethylether, saugt von den Salzen ab, engt das Filtrat im Vakuum ein und zieht dann das Dimethylformamid im Ölpumpenvakuum ab. Der Rückstand wird durch Säulenchromatographie an Kieselgel gereinigt. Als Elutionsmittel dient ein Gemisch aus Hexan und Essigester.
Ausbeute: 104,1 g (88,7 % d. Th.)

| Elementaranalyse: C₃₀H₅₂F₃N₃O₉ | | | | |
|---|---|---|---|---|
| ber. | C 54,95 | H 7,99 | F 8,69 | N 6,41 |
| gef. | C 54,99 | H 8,06 | F 8,64 | N 6,39 |

### c) 6,9-Bis(t-butoxycarbonylmethyl)-3,6, 9-triazaundecandicarbonsäure-di-t-butylester

In 400 ml Methanol werden 32,79 g (50 mmol) der unter 1b) hergestellten Verbindung gelöst. Man versetzt mit 200 ml konzentrierten Ammoniak und läßt über das Wochenende bei Raumtemperatur rühren. Das Dünnschichtchromatogramm zeigt, daß keine Ausgangsverbindung mehr vorhanden ist. Man engt im Vakuum ein, nimmt in Dichlormethan auf, wäscht mit Wasser, trocknet die organische Lösung über Natriumsulfat und engt im Vakuum zur Trockne ein. Die Titelverbindung wird als viskoses Öl erhalten.
Ausbeute: 26,64 g (95, 2 % d. Th.)

| Elementaranalyse: C₂₈H₅₃N₃O₈ | | | |
|---|---|---|---|
| ber. | C 60,06 | H 9,54 | N 7,54 |
| gef. | C 60,11 | H 9,60 | N 7,49 |

### d) 3-Benzyloxycarbonylmethyl-6,9-bis(t-butoxycarbonylmethyl)-3,6,9-triazaundecandicarbonsäure-di-t-butylester

In 120 ml absolutem Dimethylformamid werden 14,0 g (25 mmol) der unter 1c) hergestellten Verbindung gelöst. Man versetzt mit 3,86 g (28 mmol) gepulvertem Kaliumcarbonat, kühlt auf 0°C und tropft dann 5,96 g (26 mmol) Bromessigsäurebenzylester dazu. Man läßt über Nacht bei Raumtemperatur rühren, gießt auf Eiswasser, nimmt in Essigester auf, trocknet die organische Lösung über Natriumsulfat und engt im Vakuum zur Trockne ein.

Die Titelverbindung wird durch Säulenchromatographie an Kieselgel gereinigt. Als Elutionsmittel dient ein Gemisch aus Hexan und Essigester. Die Titelverbindung ist ein viskoses Öl.
Ausbeute: 15,47 g (87,4 d. Th.)

| Elementaranalyse: C₃₇H₆₁N₃O₁₀ | | | |
|---|---|---|---|
| ber. | C 62,76 | H 8,68 | N 5,96 |
| gef. | C 62,81 H | 8,73 | N 5,99 |

### e) 6,9-Bis(t-butoxycarbonylmethyl)-3-carboxymethyl-3,6,9-triazaundecandicarbonsäure-di-t-butylester

In 200 ml Isopropanol werden 7,08 g (10 mmol) der unter 1d) hergestellten Verbindung gelöst. Man versetzt mit 250 mg Katalysator (Pd 20 %/C), evakuiert, belüftet mit Wasserstoff und hydriert bei Normaldruck. Es werden 224 ml Wasserstoff aufgenommen. Man filtriert vom Katalysator ab, wäscht gut mit Isopropanol nach und engt die Lösung im Vakuum zur Trockne ein. Die Titelverbindung wird als Schaum erhalten.
Ausbeute: 5,91 g (95,6 % d. Th.)

| Elementaranalyse: C₃₀H₅₅N₃O₁₀ | | | |
|---|---|---|---|
| ber. | C 58,33 | H 8,97 | N 6,80 |
| gef. | C 58,30 | H 9,00 | N 6,75 |

### f) 6,9-Bis (tert.-butoxycarbonylmethyl) -3-pentylaminocarbonylmethyl-3,6,9-triazaundecandisäure-di-tert.-butylester

5,00 g (7,91 mmol) der Titelverbindung aus Beispiel le) werden in 25 ml Dimethylformamid gelöst und es werden 894 mg (7,77 mol) N-Hydroxysuccinimid zugegeben. Man kühlt auf 0°C ab und gibt 1,603 g (7,77 mmol) Dicyclohexylcarbodiimid hinzu. Es wird eine Stunde bei 0°C und anschließend 4 Stunden bei Raumtemperatur gerührt. Man kühlt auf 0°C ab und tropft innerhalb von 10 Minuten eine Lösung aus 0,62 g Pentylamin (7,06 mmol) in 10 ml Dimethylformamid hinzu. Man rührt eine Stunde bei 0°C, dann über Nacht bei Raumtemperatur. Nach dem Abziehen des Lösungsmittels im Vakuum wird zur Trockne eingedampft und der Rückstand in 100 ml Essigsäureethylester aufgenommen. Man filtriert vom ausgefallenem Harnstoff ab und wäscht das Filtrat 2 mal mit je 100 ml 5 %iger aqu. Soda-Lösung. Die organische Phase wird über Magnesiumsulfat getrocknet und im Vakuum zur Trockne eingedampft. Der Rückstand wird an Kieselgel chromatographiert (Laufmittel: n-
Hexan/Essigsäureethylester 20:1). Man erhält 4,81 g der Titelverbindung (88 % d. Th.) als farbloses Öl.

| Elementaranalyse: | | | |
|---|---|---|---|
| ber. | C 64,92 | H 9,34 | N 7,21 |
| gef. | C 64,81 H | 9,28 | N 7,25 |

### g) 6,9-Bis-(carboxymethyl)-3-pentylaminocarbonylmethyl-3,6,9-triazaundecandisäure

4,50 g (5,79 mmol) der Titelverbindung aus Beispiel 1f) werden in 100 ml Trifluoressigsäure gelöst. Man rührt über Nacht bei Raumtemperatur. Man dampft im Vakuum zur Trockne ein und chromatographiert den Rückstand an Kieselgel (Laufmittel: Ethanol/25 % aqu. Ammoniak-Lsung 20:1). Die das Produkt enthaltenden Fraktionen werden im Vakuum zur Trockne eingedampft. Der Rückstand wird in 100 ml Wasser gelöst. Man gibt 20 ml sauren Ionenaustauscher IR 120 (H+-Form) zu und rührt 10 Minuten bei Raumtemperatur. Man filtriert vom Ionenaustauscher ab und dampft im Vakuum zur Trockne ein.
Ausbeute: 2,35g (69 % d. Th.) eines glasigen Feststoffes Wassergehalt: 3.44 %

| Elementaranalyse (bezogen auf wasserfreie Substanz): | | | |
|---|---|---|---|
| ber. | C 56,51 H | 7,30 | N 10,14 |
| gef. | C 56,61 H | 7,22 | N 10,03 |

### h) Gadoliniumkomplex von 6,9-Bis(carboxymethyl)-3-pentylaminocarbonylmethyl-3,6,9-triazaundecandisäure-mono-Natriumsalz

2.5g (4.52 mmol) der Titelverbindung aus Beispiel 1g)werden in 75 ml destilliertem Wasser gelöst und bei Raumtemperatur portionsweise mit insgesamt 1.65 g (4.57 mmol) Gadoliniumoxid versetzt. Nach einer Reaktionszeit von 3 Stunden bei 80°C wird die nun klare Reaktionslösung auf Raumtemperatur abgekühlt und ein pH-Wert von 7,2 eingestellt. Nach Filtration über PTFE-Filter wird das so erhaltene Filtrat gefriergetrocknet.
Ausbeute: g.48 g (69 % d. Th.) eines amorphen Pulvers Wassergehalt: 3.59%

| Elementaranalyse (bezogen auf wasserfreie Substanz): | | | | | |
|---|---|---|---|---|---|
| ber. | C 41,54 | H 5,86 | N 7,75 | Gd 21,75 | Na 3,18 |
| gef. | C 41,62 | H 5,91 | N 7,71 | Gd 21,92 | Na 3,25 |

### Beispiel 2

### 6,9-Bis(t-butoxycarbonylmethyl)-3-carboxymethyl-3,6,9-triazaundecandicarbonsäure-di-t-butylester

### a) 1-Benzyl-1,4,7-triazaheptan

Eine gerührte Lösung von 108,26 ml (998 mmol) 1,4,7-Triazaheptan in 750 absolutem Methanol wird bei 0°C tropfenweise mit einer Lösung von 20,0 g (188 mmol) Benzaldehyd in 100 ml absolutem Methanol versetzt. Nach einer Reaktionszeit von 2 Stunden bei 0°C wird portionsweise mit insgesamt 7,13 g (188 mmol) Natriumborhydrid versetzt und weitere 12 Stunden bei 25°C gerührt. Zur Aufarbeitung wird das Reaktionsgemisch über Kieselgur filtriert und das Lösungsmittel im Vakuum abgezogen. Der verbleibende ölige Rückstand wird mit Dichlormethan (250 ml) und Wasser (250 ml) versetzt und extrahiert. Nach erneuter Extraktion der wässrigen Phase mit Dichlormethan werden die vereinigten organischen Phasen über Natriumsulfat getrocknet, filtriert und das Lösungsmittel im Vakuum abgezogen. Der Rückstand wird an Kieselgel chromatographiert (Eluent: Methanol/wässrige Ammoniaklösung 20/1).
Ausbeute: 31,52 g (86,7 % d. Th.) eines farblosen Öls

| Elementaranalyse: C₁₁H₁₉N₃ | | | |
|---|---|---|---|
| ber. | C 68,35 | H 9,91 | N 21,74 |
| gef. | C 67,96 | H 9,88 | N 21,70 |

### b) 6,9-Bis(t-butyloxycarbonylmethyl)-3-benzyl-3,6,9-triazaundecandicarbonsäure-di-t-butylester

19,54 g (101,1 mmol) der unter 2a) hergestellten Titelverbindung werden in einem Gemisch aus 400 ml Tetrahydrofuran und 80 ml Wasser gelöst und mit 64,3 g (465,15 mmol) Kaliumcarbonat versetzt. Anschließend tropft man 90,75 g (465,15 mmol) Bromessigsäure-t-butylester hinzu und kocht die Reaktionslösung für 3 Stunden am Rückfluß. Nach dem Abkühlen auf Raumtemperatur wird die wässrige Phase abgetrennt und noch einmal mit 200 ml Essigsäureethylester extrahiert. Die vereinigten organischen Phasen werden einmal mit gesättigter Natriumchloridlösung gewaschen und über Natriumsulfat getrocknet. Es wird filtriert und das Lösungsmittel im Vakuum vollständig abgezogen. Das zurückbleibende Öl wird säulenchromatographisch gereinigt (Eluent: Hexan/Essigsäureethylester 1:1).
Ausbeute: 48,8 g (74,4 % d. Th.)

| Elementaranalyse: C₃₅H₅₉N₃O₈ | | | |
|---|---|---|---|
| ber. | C 64, 69 | H 9,15 | N 6,47 |
| gef. | C 65,10 | H 9,21 | N 6,50 |

### c) 6,9-Bis(t-butoxycarbönylmethyl)-3,6,9-triazaundecandicarbonsäure-di-t-butylester

In 150 ml 2-Propanol werden 12,55 g (19,31 mmol) der unter 2b] hergestellten Verbindung gelöst. Man versetzt mit 1,0 g Katalysator (Pd 20 %/C), evakuiert, belüftet mit Wasserstoff und hydriert bei Normaldruck . Nach einer Reaktionszeit von 5 Stunden bei 25°C ist nach Dünnschichtchromatographie kein Ausgangsmaterial mehr erkennbar. Man filtriert vom Katalysator ab, wäscht gut mit 2-Propanol nach und engt die Lösung im Vakuum zur Trockne ein.
Ausbeute: 10,57 g (97,7 % d. Th.) als farbloses und viskoses Öl

| Elementaranalyse: C₂₈H₅₃N₃O₈ | | | |
|---|---|---|---|
| ber. | C 60,06 | H 9,54 | N 7,54 |
| gef. | C 60,13 | H 9,58 | N 7,50 |

### d) 3-Benzyloxycarbonylmethyl-6,9-bis(t-butoxycarbonylmethyl)-3, 6, 9-triazaundecandicarbonsäure-di-t-butylester

In Analogie zu Beispiel ld) liefert die Umsetzung von 10,46 g (18,69 mmol) der Titelverbindung aus Beispiel 2c) mit 2,76 g (20 mmol) Kaliumcarbonat und 4,77 g (20 mmol) Bromessigsäurebenzylester 11,6 g (88,2 % d. Th.) der Titelverbindung als viskoses Öl

| Elementaranalyse: C₃₇H₆₁N₃O₁₀ | | | |
|---|---|---|---|
| ber. | C 62,76 | H 8,68 | N 5,96 |
| gef. | C 62,79 | H 8,72 | N 5,60 |

### e) 6,9-Bis(t-butoxycarbonylmethyl)-3-carboxymethyl-3,6,9-triazaundecandicarbonsäure-di-t-butylester

In Analogie zu Beispiel le) liefert die Hydrogenolyse von 9,74 g (13,76 mmol) der Titelverbindung aus Beispiel 2d) unter Verwendung von 300 mg Katalysator (Pd 20 %/C) die Titelverbindung als farblosen Schaum.
Ausbeute: 8,17 g (96,2 % d. Th.)

| Elementaranalyse: C₃₀H₅₅N₃O₁₀ | | | |
|---|---|---|---|
| ber. | C 58,33 | H 8,97 | N 6,80 |
| gef. | C 58,29 | H 8,99 | N 6,74 |

### Beispiel 3

### 3-Carboxymethyl-6-t-butoxycarbonylmethyl-3,6-diaza-octan-dicarbonsäure-di-t-butylester

### a) 1-Benzyl-1,4-diazabutan

60,0 g (998 mmol) 1,4-Diazabutan werden in 750 ml absolutem Methanol gelöst und auf 0°C gekühlt. Bei dieser Temperatur erfolgt die tropfenweise Addition von 20,0 g (188 mmol) Benzaldehyd, gelöst in 100 ml absolutem Methanol. Nach einer Reaktionszeit von 2 Stunden bei 0°C erfolgt die portionsweise Zugabe von insgesamt 7,13 g (188 mmol) Natriumborhydrid. Nach 12 Stunden bei 25°C wird die Reaktionslösung über Kieselgur abgesaugt und das Lösungsmittel im Vakuum abgezogen. Der zurückbleibende ölige Rückstand wird in 500 ml Dichlormethan aufgenommen und dreimal mit jeweils 100 ml Wasser gewaschen. Nach dem Trocknen der organischen Phase über Natriumsulfat wird das Lösungsmittel im Vakuum abgezogen. Der so erhaltene ölige Rückstand wird an Kieselgel chromatographiert (Eluent: Methanol/wässrige Ammoniaklösung 20:1).
Ausbeute: 24,2 g (85,7 % d. Th.)

| Elementaranalyse: C₉H₁₄N₂ | | | |
|---|---|---|---|
| ber. | C 71,96 | H 9,39 | N 18,65 |
| gef. | C 71,89 | H 9,37 | N 18,60 |

### b) 3-Benzyl-6-t-butyloxycarbonylmethyl-3,6-diazaoctandicarbonsäure-di-t-butylester

15,04 g (100,11 mmol) der unter 3a) hergestellten Titelverbindung werden in einem Gemisch aus 400 ml Tetrahydrofuran und 80 ml Wasser gelöst und mit 49,76 g (360 mmol) Kaliumcarbonat versetzt. Anschließend tropft man 70,22 g (360 mmol) Bromessigsäure-t-butylester hinzu und kocht die Reaktionslösung 3 Stunden am Rückfluß. Nach dem Abkühlen auf Raumtemperatur wird die wässrige Phase abgetrennt und noch einmal mit 200 ml Essigsäureethylester extrahiert. Die vereinigten organischen Phasen werden einmal mit gesättigter Natriumchloridlösung gewaschen und über Natriumsulfat getrocknet. Es wird filtriert und das Lösungsmittel im Vakuum vollständig abgezogen. Das zurückbleibende Öl wird säulenchromatographisch gereinigt (Eluent: Hexan/Essigsäureethylester 1:1).
Ausbeute: 47,15 g (95,6 % d. Th.) als farbloses Öl

| Elementaranalyse: C₂₇H₄₄N₂O₆ | | | |
|---|---|---|---|
| ber. | C 65,83 | H 9,00 | N 5,69 |
| gef. | C 65,78 | H 8,97 | N 5,68 |

### c) 6-t-Butoxycarbonylmethyl-3,6-diazaoctandicarbonsäure-di-t-butylester

In 250 ml 2-Propanol werden 31,11 g (63,15 mmol) der unter 3b) hergestellten Verbindung gelöst. Man versetzt mit 1,0 g Katalysator (Pd 20 %/C), evakuiert, belüftet mit Wasserstoff und hydriert bei Normaldruck . Nach einer Reaktionszeit von 5 Stunden bei 25°C ist nach Dünnschichtchromatographie kein Ausgangsmaterial mehr erkennbar. Man filtriert vom Katalysator ab, wäscht gut mit 2-Propanol nach und engt die Lösung im Vakuum zur Trockne ein.
Ausbeute: 20,07 g (78,95 % d. Th.) als farbloses Öl

| Elementaranalyse: C₂₀H₃₈N₂O₆ | | | |
|---|---|---|---|
| ber. | C 59,68 | H 9,51 | N 6,96 |
| gef. | C 59,62 | H 9,49 | N 6,97 |

### d) 3-Benzyloxycarbonylmethyl-6-t-butoxycarbonylmethyl-3,6-diazaoctandicarbonsäure-di-t-butylester

6,0 g (14,91 mmol) der unter 3c) hergestellten Titelverbindung werden in einem Gemisch aus 125 ml Tetrahydrofuran und 25 ml Wasser gelöst und mit 2,68 g (19,38 mmol) Kaliumcarbonat versetzt. Anschließend tropft man 4,44 g (19,38 mmol) Bromessigsäurebenzylester hinzu und kocht die Reaktionslösung für 3 Stunden am Rückfluß. Nach dem Abkühlen auf Raumtemperatur wird die wässrige Phase abgetrennt und noch einmal mit 200 ml Essigsäureethylester extrahiert. Die vereinigten organischen Phasen werden einmal mit gesättigter Natriumchloridlösung gewaschen und über Natriumsulfat getrocknet. Es wird filtriert und das Lösungsmittel im Vakuum vollständig abgezogen. Das zurückbleibende Öl wird säulenchromatographisch gereinigt (Eluent: Hexan/Essigsäureethylester 1:1).
Ausbeute: 6,76 g (82,4 % d. Th.) als farbloses Öl

| Elementaranalyse: C₂₉H₄₆N₂O₈ | | | |
|---|---|---|---|
| ber. | C 63,25 | H 8,42 | N 5,09 |
| gef. | C 63,31 H | 8,44 | N 5,13 |

### e) 3-Carboxymethyl-6-t-butoxycarbonylmethyl-3,6-diazaoctandicarbonsäure-di-t-butylester

4,8 g (8,72 mmol) der Titelverbindung aus Beispiel 3d) werden in 50 ml 2-Propanol gelöst und mit 150 mg Katalysator (20 % Pd/C) versetzt. Es wird evakuiert, mit Wasserstoff belüftet und bei Normaldruck 6 Stunden hydriert. Zur Aufarbeitung wird vom Katalysator abfiltriert und gut mit 2-Propanol nachgewaschen. Das Filtrat wird im bis zur Trockne eingeengt. Säulenchromatographische Reinigung des verbleibenden Rückstandes an Kieselgel (Eluent: Dichlormethan/2-Propanol 10:1) liefert die Titelverbindung als farbloses Öl.
Ausbeute: 3,4 g ( 85 % d. Th.)

| Elementaranalyse: C₂₂H₄₀N₂O₈ | | | |
|---|---|---|---|
| ber. | C 57,37 | H 8,75 | N 6,08 |
| gef. | C 57,29 | H 8,71 | N 6,04 |

### Beispiel 4

### (±)-trans-N,N',N'-tri-t-butoxycarbonylmethyl-N-carboxymethyl-1, 2-diaminocyclohexan

### a) (±) -trans-N-Benzyl-1, 2-diaminocyclohexan

60,0 g (525,4 mmol) (±)-trans-1,2-Diaminocyclohexan werden in 750 ml absolutem Methanol gelöst und auf 0°C gekühlt. Bei dieser Temperatur erfolgt die tropfenweise Zugabe von 10,5 g (99 mmol) Benzaldehyd, gelöst in 100 ml absolutem Methanol. Nach einer Reaktionszeit von 2 Stunden bei 0°C erfolgt die portionsweise Zugabe von insgesamt 3,74 g (99 mmol) Natriumborhydrid. Nach 12 Stunden bei 25°C wird die Reaktionslösung über Kieselgur abgesaugt und das Lösungsmittel im Vakuum abgezogen. Der zurückbleibende ölige Rückstand wird in 500 ml Dichlormethan aufgenommen und dreimal mit jeweils 100 ml Wasser gewaschen. Nach dem Trocknen der organischen Phase über Natriumsulfat wird das Lösungsmittel im Vakuum abgezogen. Der so erhaltene ölige Rückstand wird an Kieselgel chromatographiert (Eluent: Methanol/wässrige Ammoniaklösung 20:1).
Ausbeute: 93,1 g (86,8 % d. Th.)

| Elementaranalyse: C₁₃H₂₀N₂ | | | |
|---|---|---|---|
| ber. | C 76,42 | H 9,87 | N 13,71 |
| gef. | C 76,40 | H 9,88 | N 13,69 |

b) (±)-trans-N-Benzyl-N,N',N'-tri-t-butoxycarbonylmethyl-1,2-diaminocyclohexan

80 g (391,56 mmol) der unter 4a) hergestellten Titelverbindung werden in einem Gemisch aus 1000 ml Tetrahydrofuran und 250 ml Wasser gelöst und mit 195 g (1409,6 mmol) Kaliumcarbonat versetzt. Anschließend tropft man 275 g (1409,6 mmol) Bromessigsäure-t-butylester hinzu und kocht die Reaktionslösung für 3 Stunden am Rückfluß. Nach dem Abkühlen auf Raumtemperatur wird die wässrige Phase abgetrennt und noch einmal mit 600 ml Essigsäureethylester extrahiert. Die vereinigten organischen Phasen werden einmal mit gesättigter Natriumchloridlösung gewaschen und über Natriumsulfat getrocknet. Es wird filtriert und das Lösungsmittel im Vakuum vollständig abgezogen. Das zurückbleibende Öl wird säulenchromatographisch gereinigt (Eluent: Hexan/Essigsäureethylester 1:1).
Ausbeute: 197,81 (92,4 % d. Th.) als farbloses Öl

| Elementaranalyse: C₃₁H₅₀N₂O₆ | | | |
|---|---|---|---|
| ber. | C 68,10 | H 9,22 | N 5,12 |
| gef. | C 68,07 | H 9,20 | N 5,09 |

### c) (±)-trans-N,N',N'-tri-t-butoxycarbonylmethyl-1,2-diaminocyclohexan

In 250 ml 2-Propanol werden 30,0 g (54,87 mmol) der unter 4b) hergestellten Verbindung gelöst. Man versetzt mit 1,0 g Katalysator (Pd 20 %/C), evakuiert, belüftet mit Wasserstoff und hydriert bei Normaldruck . Nach einer Reaktionszeit von 5 Stunden bei 25°C ist nach Dünnschichtchromatographie kein Ausgangsmaterial mehr erkennbar. Man filtriert vom Katalysator ab, wäscht gut mit 2-Propanol nach und engt die Lösung im Vakuum zur Trockne ein.
Ausbeute: 20,8 g (83,2 % d. Th.) als farbloses Öl

| Elementaranalyse: C₂₄H₄₄N₂O₆ | | | |
|---|---|---|---|
| ber. | C 63,13 | H 9,71 | N 6,13 |
| gef. | C 63,11 | H 9,69 | N 6,11 |

### d) (±)-trans-N,N',N'-tri-t-butoxycarbonylmethyl-N-benzyloxycarbonylmethyl-1,2-diaminocyclohexan

5,0 g (10,95 mmol) der unter 4c) hergestellten Titelverbindung werden in einem Gemisch aus 125 ml Tetrahydrofuran und 25 ml Wasser gelöst und mit 1,96 g (14,23 mmol) Kaliumcarbonat versetzt. Anschließend tropft man 3,2 g (14,23 mmol) Bromessigsäurebenzylester hinzu und kocht die Reaktionslösung für 3 Stunden am Rückfluß. Nach dem Abkühlen auf Raumtemperatur wird die wässrige Phase abgetrennt und noch einmal mit 200 ml Essigsäureethylester extrahiert. Die vereinigten organischen Phasen werden einmal mit gesättigter Natriumchloridlösung gewaschen und über Natriumsulfat getrocknet. Es wird filtriert und das Lösungsmittel im Vakuum vollständig abgezogen. Das zurückbleibende Öl wird säulenchromatographisch gereinigt (Eluent: Hexan/Essigsäureethylester 1:1).
Ausbeute: 5,52 g (83,5 % d. Th.) als farbloses Öl

| Elementaranalyse: C₃₃H₅₂N₂O₈ | | | |
|---|---|---|---|
| ber. | C 65,54 | H 8,67 | N 4,63 |
| gef. | C 65,49 | H 8,63 | N 4,60 |

### e) (±)-trans-N,N',N'-tri-t-butoxycarbonylmethyl-N-carboxymethyl-1,2-diaminocyclohexan

4,0 g (6,60 mmol) der Titelverbindung aus Beispiel 4d) werden in 50 ml 2-Propanol gelöst und mit 150 mg Katalysator (20 % Pd/C) versetzt. Es wird evakuiert, mit Wasserstoff belüftet und bei Normaldruck 6 Stunden hydriert. Zur Aufarbeitung wird vom Katalysator abfiltriert und gut mit 2-Propanol nachgewaschen. Das Filtrat wird im bis zur Trockne eingeengt. Säulenchromatographische Reinigung des verbleibenden Rückstandes an Kieselgel (Eluent: Dichlormethan/2-Propanol 10:1) liefert die Titelverbindung als farbloses Öl.
Ausbeute: 3,0 g (88,2 % d. Th.)

| Elementaranalyse: C₂₆H₄₆N₂O₈ | | | |
|---|---|---|---|
| ber. | C 60,68 | H 9,01 | N 5,44 |
| gef. | C 60,70 | H 9,03 | N 5,47 |

## Patentansprüche

1. Verbindungen der allgemeinen Formel I 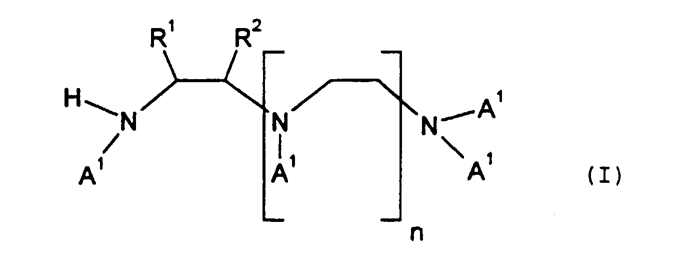 worin
n für die Zahlenwerte 0, 1 oder 2 steht,
A¹ für den Rest -CH₂CO₂^{t}Bu steht,
R¹ und R² entweder jeweils für Wasserstoff oder gemeinsam für -(CH₂)ₘ- stehen, worin m die Zahlenwerte 3 bis 6 annehmen kann,
mit der Maßgabe, daß R¹ und R² nur dann gemeinsam für -(CH₂)ₘ- stehen, wenn n für den Wert 0 steht.

2. Verbindungen der allgemeinen Formel II 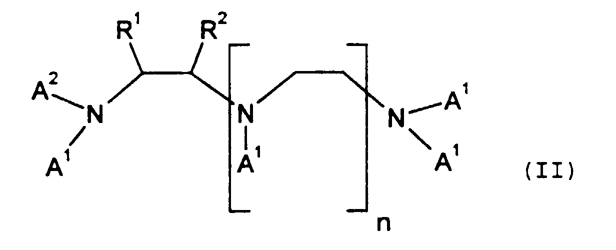
A¹ für den Rest -CH₂CO₂^{t}Bu steht,
A² für den Rest -CH₂CO₂H steht,
R¹ und R² entweder jeweils für Wasserstoff oder gemeinsam für -(CH₂)ₘ- steht, worin m die Zahlenwerte 3 bis 6 annehmen kann,
mit der Maßgabe, daß R¹ und R² nur dann gemeinsam für -(CH₂)ₘ- stehen, wenn n für den Wert 0 steht.

3. Verbindungen gemäß Anspruch 1 oder 2, dadurch gekennzeichnet, daß n für die Zahl 1 steht.

4. Verbindungen gemäß Anspruch 1 oder 2, dadurch gekennzeichnet, daß R¹ und R² für Wasserstoff stehen.

5. Verbindungen gemäß Anspruch 1 oder 2, dadurch gekennzeichnet, daß R¹ und R² gemeinsam für -(CH₂)₄- stehen.

6. Verfahren zur Herstellung von Verbindungen der allgemeinen Formel I 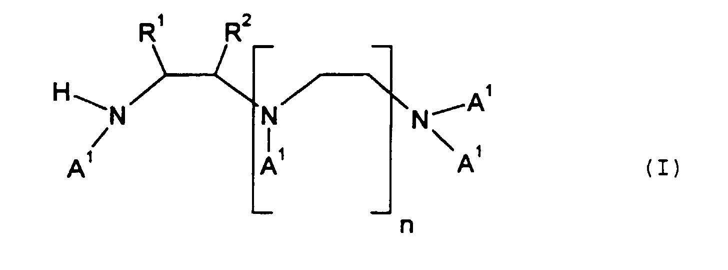 worin
n für die Zahlenwerte 0, 1 oder 2 steht,
A¹ für den Rest -CH₂CO₂^{t}Bu steht,
R¹ und R² entweder für Wasserstoff oder gemeinsam für -(CH₂)ₘ- steht, worin m die Zahlenwerte 3 bis 6 annehmen kann,
mit der Maßgabe, daß R¹ und R² nur dann gemeinsam für -(CH₂)ₘ- stehen, wenn n für den Wert 0 steht,
dadurch gekennzeichnet daß, eine Verbindung der allgemeinen Formel III 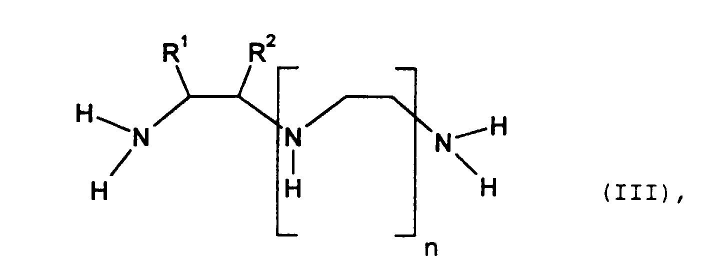 worin R¹, R² und n die oben genannten Bedeutungen besitzen mit einem Schutzgruppenreagenz zu einer Verbindung der allgemeinen Formel IV 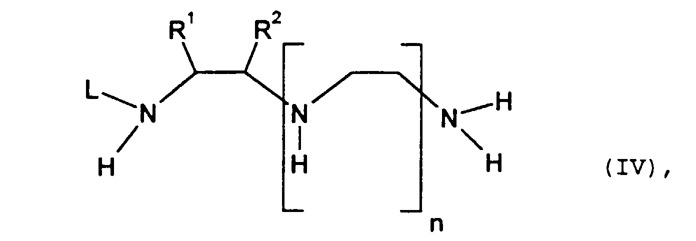 worin
R¹, R² und n die oben genannten Bedeutungen besitzen und
L für eine Trifluoracetyl- oder eine Benzylgruppe steht
umgesetzt wird, diese mit Verbindungen der allgemeinen Formel V
X-CH₂-COO^{*t*}Bu (V)
worin X für Chlor, Brom oder Jod steht,
umgesetzt wird
und die so erhaltene Verbindung durch Abspaltung der Schutzgruppe L in die Verbindung der allgemeinen Formel I überführt wird.

7. Verwendung von Verbindungen der allgemeinen Formel I, 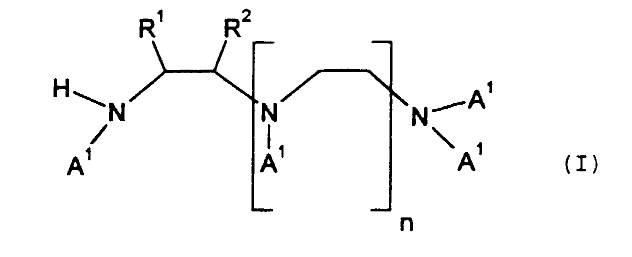 worin
n für die Zahlenwerte 0, 1 oder 2 steht,
A¹ für den Rest -CH₂CO₂^{t}Bu steht,
R¹ und R² entweder für Wasserstoff oder gemeinsam für -(CH₂)ₘ- steht, worin m die Zahlenwerte 3 bis 6 annehmen kann,
mit der Maßgabe, daß R¹ und R² nur dann gemeinsam für -(CH₂)ₘ- stehen, wenn n für den Wert 0 steht,
für die Herstellung von Mitteln für die medizinische Diagnostik oder Therapie.

8. Verwendung von Verbindungen der allgemeinen Formel II 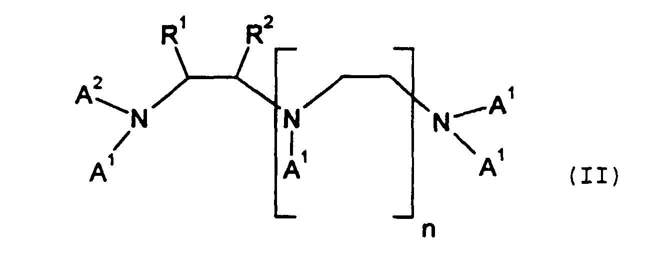 worin
n für die Zahlenwerte 0, 1 oder 2 steht,
A¹ für den Rest -CH₂CO₂^{t}Bu steht,
A² für den Rest -CH₂CO₂H steht,
R¹ und R² entweder für Wasserstoff oder gemeinsam für -(CH₂)ₘ- steht, worin m die Zahlenwerte 3 bis 6 annehmen kann,
mit der Maßgabe, daß R¹ und R² nur dann gemeinsam für -(CH₂)ₘ- stehen, wenn n für den Wert 0 steht,
für die Herstellung von Mitteln für die medizinische Diagnostik oder Therapie.

9. Verwendung gemäß Anspruch 7 oder 8 für die Herstellung von Mitteln für die MRI-Diagnostik, Röntgendiagnostik oder Radiodiagnostik.

10. Verwendung gemäß Anspruch 7 oder 8 für die Herstellung von Mitteln für die Radiotherapie.

11. Verwendung gemäß Anspruch 7 oder 8 für die Herstellung von Mitteln die als Antidot bei Schwermetallvergiftungen wirken.

## Claims

1. Compounds of the general formula I 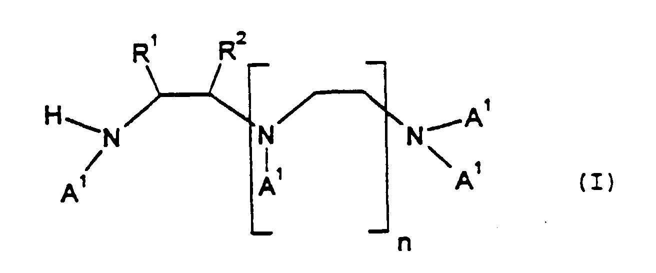 in which
n is the numerical value 0, 1 or 2,
A¹ is the radical -CH₂CO₂^{t}Bu,
R¹ and R² are either each hydrogen or together are -(CH₂)ₘ-, in which m can assume the numerical values 3 to 6,
with the proviso that R¹ and R² are only jointly -(CH₂)ₘ- when n is the value 0.

2. Compounds of the general formula II 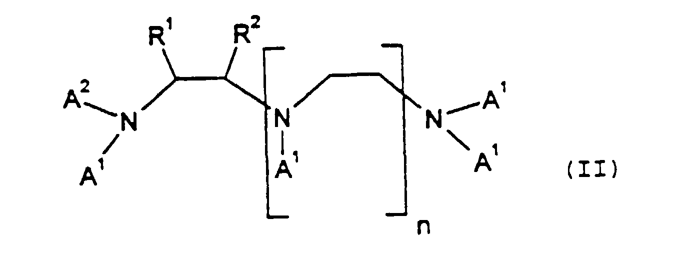 in which
n is the numerical value 0, 1 or 2,
A¹ is the radical -CH₂CO₂^{t}Bu,
A² is the radical -CH₂CO₂H,
R¹ and R² are either each hydrogen or together are -(CH₂)ₘ-, in which m can assume the numerical values 3 to 6,
with the proviso that R¹ and R² are only jointly -(CH₂)ₘ- when n is the value 0.

3. Compounds according to Claim 1 or 2, characterized in that n is the number 1.

4. Compounds according to Claim 1 or 2, characterized in that R¹ and R² are hydrogen.

5. Compounds according to Claim 1 or 2, characterized in that R¹ and R² together are -(CH₂)₄-.

6. Process for the preparation of compounds of the general formula I 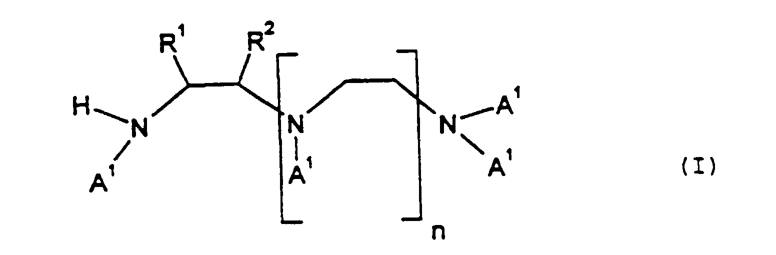 in which
n is the numerical value 0, 1 or 2,
A¹ is the radical -CH₂CO₂^{t}Bu,
R¹ and R² are either each hydrogen or together are -(CH₂)ₘ-, in which m can assume the numerical values 3 to 6,
with the proviso that R¹ and R² are only jointly -(CH₂)ₘ- when n is the value 0,
characterized in that a compound of the general formula III 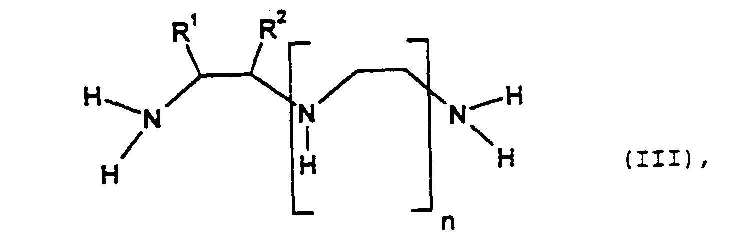 in which R¹, R² and n have the abovementioned meanings, is reacted with a protective group reagent to give a compound of the general formula IV 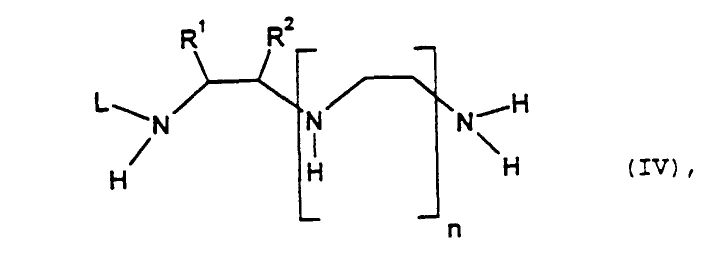
in which R¹, R² and n have the abovementioned meanings and
L is a trifluoroacetyl or a benzyl group,
this is reacted with compounds of the general formula V
X-CH₂-COO^{t}Bu (V)
in which X is chlorine, bromine or iodine,
and the compound thus obtained is converted into the compound of the general formula I by removal of the protective group L.

7. Use of compounds of the general formula I 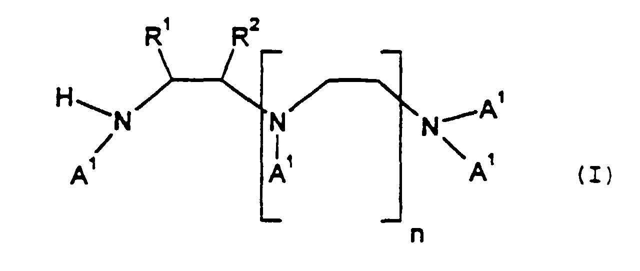 in which
n is the numerical value 0, 1 or 2,
A¹ is the radical -CH₂CO₂^{t}Bu,
R¹ and R² are either each hydrogen or together are -(CH₂)ₘ-, in which m can assume the numerical values 3 to 6,
with the proviso that R¹ and R² are only jointly -(CH₂)ₘ- when n is the value 0,
for the preparation of agents for medical diagnosis or therapy.

8. Use of compounds of the general formula II 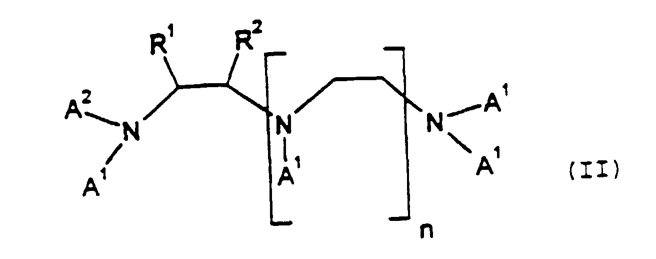 in which
n is the numerical value 0, 1 or 2,
A¹ is the radical -CH₂CO₂^{t}Bu,
A² is the radical -CH₂CO₂H,
R¹ and R² are either each hydrogen or together are -(CH₂)ₘ-, in which m can assume the numerical values 3 to 6,
with the proviso that R¹ and R² are only jointly -(CH₂)ₘ- when n is the value 0,
for the preparation of agents for medical diagnosis or therapy.

9. Use according to Claim 7 or 8 for the preparation of agents for MRI diagnosis, X-ray diagnosis or radiodiagnosis.

10. Use according to Claim 7 or 8 for the preparation of agents for radiotherapy.

11. Use according to Claim 7 or 8 for the preparation of agents which act as an antidote in heavy metal poisoning.

## Revendications

1. Composés de formule générale I 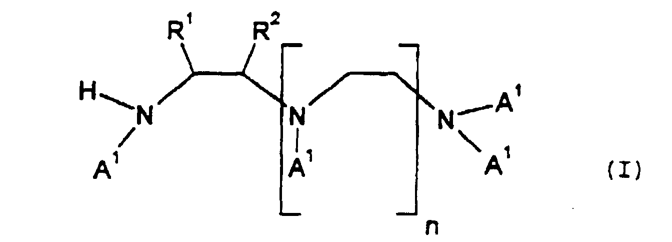 dans laquelle,
n signifie les valeurs numériques 0, 1 ou 2,
A¹signifie le radical -CH₂CO₂^{t}Bu,
R¹ et R² signifient soit chacun de l'hydrogène soit ensemble (CH₂)ₘ-, dans lequel m peut prendre les valeurs numériques 3 à 6,
étant entendu que R¹ et R² ne signifient (CH₂)ₘ-ensemble que lorsque n signifie la valeur 0.

2. Composés de formule générale II 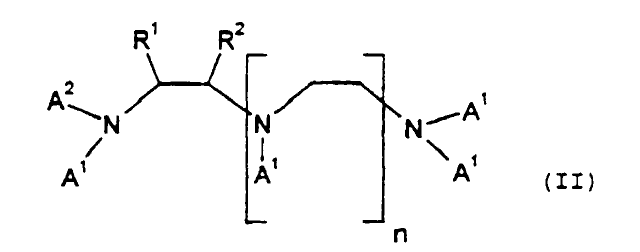 dans laquelle
n signifie les valeurs numériques 0,1 ou 2
A¹ signifie le radical -CH₂CO₂^{t}Bu,
A² signifie le radical -CH₂CO₂H,
R¹ et R² signifient soit chacun de l'hydrogène, soit ensemble (CH₂)ₘ-, dans lequel m peut prendre les valeurs numériques 3 à 6,
étant entendu que R¹ et R² ne signifient (CH₂)ₘ-ensemble que lorsque n signifie la valeur 0.

3. Composés suivant la revendication 1 ou 2, caractérisés en ce que n signifie le nombre 1.

4. Composés suivant la revendication 1 ou 2, caractérisés en ce que R¹ et R² signifient de l'hydrogène.

5. Composés suivant la revendication 1 ou 2, caractérisés en ce que R¹ et R² signifient ensemble -(CH₂)₄-.

6. Procédé pour la préparation de composés de formule générale I 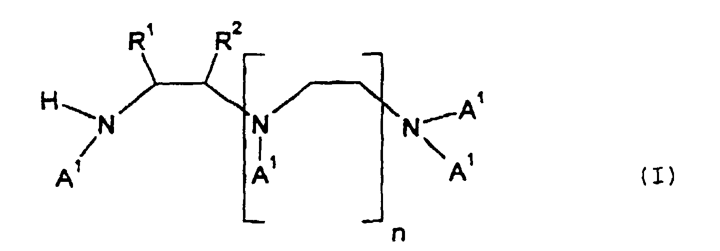 dans laquelle
n signifie les valeurs numériques 0, 1 ou 2,
A¹ signifie le radical -CH₂CO₂^{t}Bu-,
R¹ et R²signifient soit chacun de l'hydrogène, soit ensemble (CH₂)ₘ-, dans laquelle m peut prendre les valeurs numériques 3 à 6,
étant entendu que R¹ et R² ne signifient (CH₂)ₘ-ensemble que lorsque n signifie la valeur 0,
caractérisé en ce qu'un composé de formule générale III 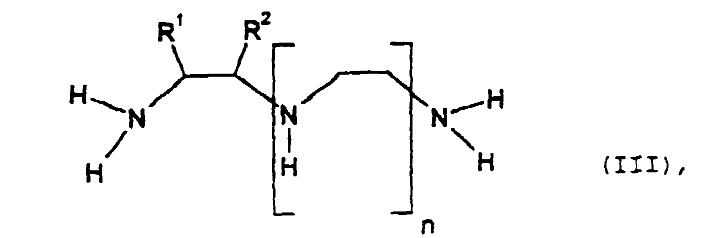 dans laquelle R¹, R² et n possèdent les significations susmentionnées est transformé avec un réactif à groupements protecteurs en un composé de formule générale IV 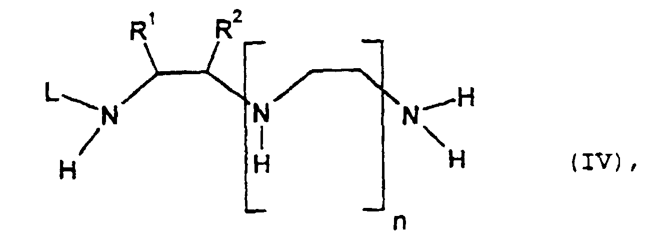 dans laquelle
R¹, R² et n possèdent les significations susmentionnées et
L signifie un groupement trifluoroacétyle ou un groupement benzyle,
en ce que celui-ci est transformé avec des composés de formule générale V
X-CH₂-COO^{t}Bu (V)
dans laquelle X signifie du chlore, du brome ou de l'iode,
et en ce que le composé ainsi obtenu est transformé par séparation du groupement protecteur L en le composé de formule générale I.

7. Utilisation de composés de formule générale I, 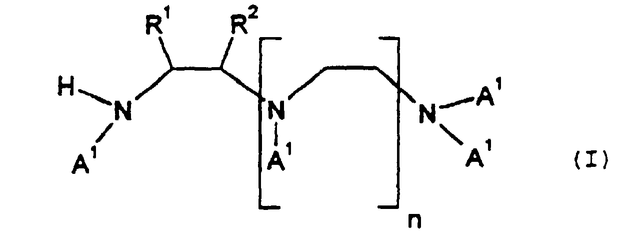 dans laquelle
n signifie les valeurs numériques 0, 1 ou 2,
A¹ signifie le radical -CH₂CO₂^{t}Bu-,
R¹ et R²signifient soit chacun de l'hydrogène, soit ensemble (CH₂)ₘ-, dans lequel m peut prendre les valeurs numériques 3 à 6,
étant entendu que R¹ et R² ne signifient (CH₂)ₘ-ensemble que lorsque n signifie la valeur 0,
pour la préparation d'agents pour le diagnostic médical ou la thérapie.

8. Utilisation des composés de formule générale II, 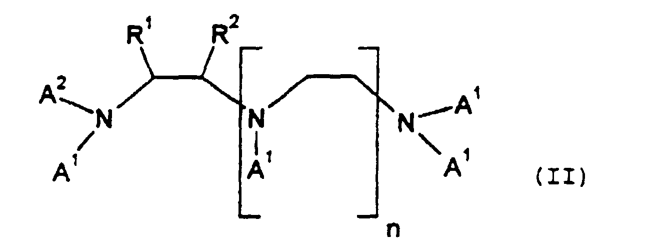 dans laquelle
n signifie les valeurs numériques 0, 1 ou 2,
A¹signifie le radical -CH₂CO₂^{t}Bu,
A² signifie le radical -CH₂CO₂H,
R¹ et R² signifient soit chacun de l'hydrogène, soit
ensemble (CH₂)ₘ-, dans lequel m peut prendre les valeurs numériques 3 à 6,
étant entendu que R¹ et R² ne signifient (CH₂)ₘ-ensemble que lorsque n signifie la valeur 0,
pour la préparation d'agents pour le diagnostic médical ou la thérapie.

9. Utilisation suivant la revendication 7 ou 8, pour la préparation d'agents pour le diagnostic IRM, le diagnostic par rayons X ou le radio-diagnostic.

10. Utilisation suivant la revendication 7 ou 8, pour la préparation d'agents pour la radiothérapie.

11. Utilisation suivant la revendication 7 ou 8, pour la préparation d'agents opérant comme antidote en cas d'intoxication aux métaux lourds.
